(19) Europäisches Patentamt
European Patent Office
Office européen des brevets

(11) Publication number: **0 045 205**
**B1**

(12) **EUROPEAN PATENT SPECIFICATION**

(45) Date of publication of patent specification: **03.10.84**

(21) Application number: **81303423.8**

(22) Date of filing: **27.07.81**

(51) Int. Cl.$^3$: **A 61 K 31/365,** C 07 H 17/08, C 12 P 19/62, C 12 P 17/08, C 07 D 313/00 // (C12P19/62, C12R1/54),(C12P17/08, C12R1/54)

# (54) Macrolides.

(30) Priority: **29.07.80 US 173313**
**29.07.80 US 173312**

(43) Date of publication of application:
**03.02.82 Bulletin 82/05**

(45) Publication of the grant of the patent:
**03.10.84 Bulletin 84/40**

(84) Designated Contracting States:
**AT BE CH DE FR GB IT LI LU NL SE**

(56) References cited:
**US-A-4 201 843**

**CHEMICAL ABSTRACTS, volume 93, no. 13, 29th September 1980, page 506, abstract 130487y COLUMBUS OHIO (US)**

(73) Proprietor: **ELI LILLY AND COMPANY**
**307, East McCarty Street**
**Indianapolis Indiana 46285 (US)**

(72) Inventor: **Seno, Eugene Thomas**
**Cottage No. 1 John Innes Institute**
**Colney Lane Norwich NR4 7UH (GB)**
Inventor: **Baltz, Richard Henry**
**7446, Sunset Lane**
**Indianapolis Indiana 46260 (US)**
Inventor: **Hamill, Robert L.**
**717 Brookview Drive**
**Greenwood Indiana 46142 (US)**
Inventor: **Wild, Gene Muriel**
**7455 Jewel Lane**
**Indianapolis Indiana 46250 (US)**

(74) Representative: **Hudson, Christopher Mark et al**
**Erl Wood Manor**
**Windlesham Surrey GU20 6PH (GB)**

**EP 0 045 205 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to a new macrolide compound and to related derivatives from which useful antibiotics, such as tylosin (see, for example, *Tetrahedron Letters,* 2339 (1970)) and tylosin derivatives, can be prepared.

This new compound, which is 5-O-mycarosyl-20-dihydro-20,23-dideoxytylonolide, will be called mycarosyltylactone for convenience herein. Mycarosyltylactone has structure *1*:

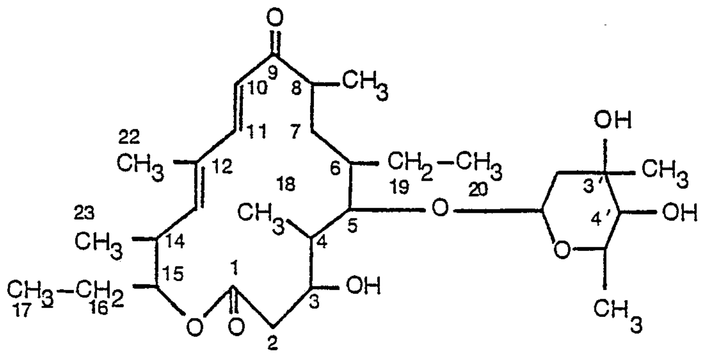

*1*

Although no stereochemical assignments are indicated in the structures given herein, the stereochemistry of the compounds is identical to that of the corresponding portion of tylosin. As the name indicates, the sugar in structure *1* is mycarose.

Related mycarosyltylactone derivatives have structure *2*:

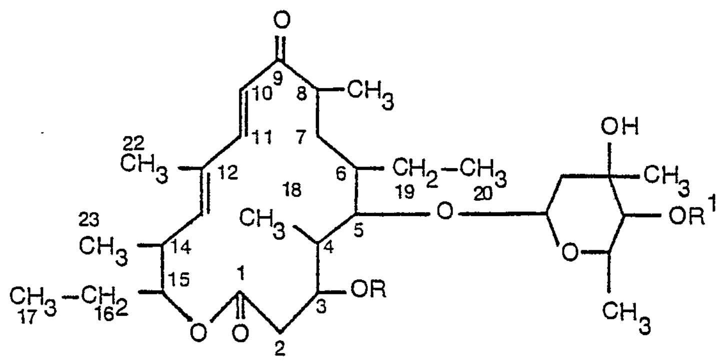

2

wherein R and R[1] are acyl moieties.

This invention further relates to 3-O-acyltylactone derivatives which can be prepared from the mycarosyltylactone derivatives of structure *2*. In another aspect, this invention provides a process for preparing tylactone or the 3-O-acyltylactone derivatives by mild acid hydrolysis of mycarosyltylactone or the mycarosyltylactone derivatives of structure *2*, respectively.

The 3-O-acyltylactone derivatives of this invention have structure *3*:

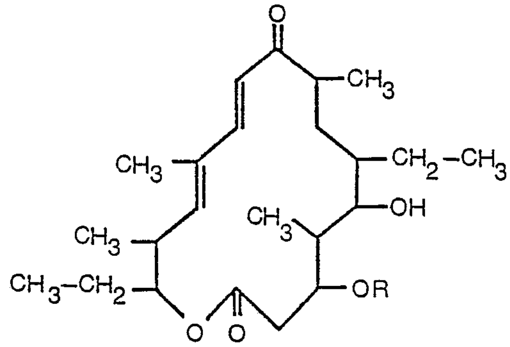

*3*

The compounds of structures *1*, *2*, and *3* are useful intermediates from which 16-membered macrolide antibiotics can be prepared. Thus, for example, this invention provides a new process for chemically derivatizing a compound of formula *3* to give the corresponding 3-monoacyl 16-membered macrolide antibiotic.

One important use of mycarosyltylactone is as an intermediate to make tylactone and tylactone derivatives. Tylactone has the formula *4*.

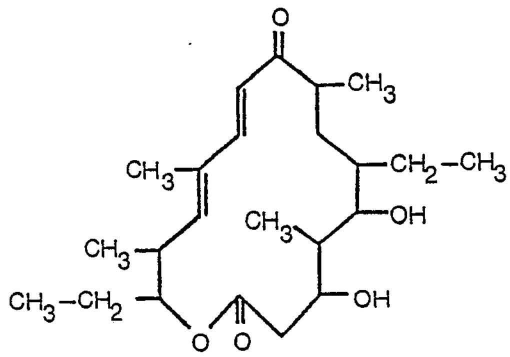

*4*

Mycarosyltylactone can be distinguished from tylactone and tylosin by silica-gel thin-layer chromatography (TLC). Sulfuric acid spray, either concentrated or diluted (50%), may be used for detection. With this detection system tylactone appears initially as a yellow-to-brown spot, and mycarosyltylactone appears as a blue-purple spot. If silica-gel plates with a fluorescent background are used in the chromatography, UV detection is convenient. The approximate Rf values of mycarosyltylactone are summarized in Table 1.

TABLE 1

TLC of Mycarosyltylactone[a]

| Compound | Rf Value | |
|---|---|---|
| | A[b] | B |
| Mycarosyltylactone | 0.17 | 0.44 |
| Tylactone | 0.50 | 0.62 |
| Tylosin | 0.0 | 0.0 |

[a]Medium: Silica gel
[b]Solvent: A = benzene: ethyl acetate (4:1)
B = benzene: ethyl acetate (3:2)

Mycarosyltylactone can be prepared by culturing a strain of *Streptomyces fradiae* which produces this compound under submerged aerobic conditions in a suitable culture medium until a substantial amount of compound is produced.

The culture medium used to grow the *Streptomyces fradiae* can be any one of a number of media. For economy in production, optimal yield, and ease of product isolation, however, certain culture media are preferred. Thus, for example, preferred carbon sources in large-scale fermentation include carbohydrates such as dextrin, glucose, starch, and corn meal and oils such as soybean oil. Preferred nitrogen sources include corn meal, soybean meal, fish meal and amino acids. Among the nutrient inorganic salts which can be incorporated in the culture media are the customary soluble salts capable of yielding for instance iron, potassium, sodium, magnesium, calcium, ammonium, chloride, carbonate, sulfate or nitrate.

Essential trace elements necessary for the growth and development of the organism should also be included in the culture medium. Such trace elements commonly occur as impurities in other constituents of the medium in amounts sufficient to meet the growth requirements of the organism. It may be necessary to add small amounts (i.e. 0.2 ml/L) of an antifoam agent such as polypropylene glycol (M.W. about 2000) to large-scale fermentation media if foaming becomes a problem.

For production of substantial quantities of mycarosyltylactone, submerged aerobic fermentation in tanks is preferred. Small quantities of mycarosyltylactone may be obtained by shake-flask culture.

Because of the time lag in production commonly associated with inoculation of large tanks with the spore form of the organism, it is preferable to use a vegetative inoculum. The vegetative inoculum is prepared by inoculating a small volume of culture medium with the spore form or mycelial fragments of the organism to obtain a fresh, actively growing culture. The vegetative inoculum is then transferred to a larger tank. The medium used for the vegetative inoculum can be the same as that used for larger fermentations, but other media can also be used.

The microorganism of use in the process of the invention was obtained by chemical mutagenesis of a *Streptomyces fradiae* strain which produced tylosin. The microorganism obtained by mutagenesis produces only minimal amounts of tylosin, but produces mycarosyltylactone and tylactone as major components.

The new microorganism which produces mycarosyltylactone is classified as a strain of *Streptomyces fradiae* . A culture of this microorganism has been deposited and made part of the stock culture collection of the Northern Regional Research Center, Agricultural Research, North Central Region, 1815 North University Street, Peoria, Illinois, 61604, from which it is available to the public under the accession number NRRL 12201.

As is the case with other organisms, the characteristics of *Streptomyces fradiae* NRRL 12201 are subject to variation. Recombinants, mutants or variants of the NRRL 12201 strain can be obtained by methods known in the art. For example mutants can be obtained by treatment with various known physical and chemical mutagens, such as ultraviolet light, X-rays, gamma rays, and N-methyl-N′-nitro-N-nitrosoguanidine. All natural and induced variants, mutants and recombinants of *Streptomyces fradiae* NRRL 12201 which retain the characteristic of mycarosyltylactone production may be used to prepare the compounds of this invention.

*S. fradiae* NRRL 12201* can be grown at temperatures between about 10° and about 40°C. Optimum production of mycarosyltylactone appears to occur at temperatures of about 28°C.

As is customary in aerobic submerged culture processes, sterile air is bubbled through the culture medium. For efficient antibiotic production the percent of air saturation for tank production should be about 30% or above (at 28°C and one atmosphere of pressure).

Production of mycarosyltylactone and tylactone can be followed during the fermentation by testing samples of the broth, using TLC or high-peformance liquid chromatography with a UV detection system.

Following its production under submerged aerobic fermentation conditions, mycarosyltylactone can be recovered from the fermentation medium by methods used in the art. Because of the limited solubility of mycarosyltylactone in water, it may not be altogether soluble in the medium in which it is produced. Recovery of mycarosyltylactone, therefore, can be accomplished by 1) extraction of the fermentation broth or 2) filtration of the fermentation broth and extraction of both the filtered broth and the mycelial cake. A variety of techniques may be used in the extraction processes. A preferred technique for purification of the filtered broth involves extracting the broth (generally without pH adjustment) with a suitable solvent such as amyl acetate or petroleum ether, concentrating the organic phase under vacuum to give crystals or an oil. The crystals or oil thus obtained may be purified by adsorption chromatography to give mycarosyltylactone and tylactone.

Mycarosyltylactone can be esterified at the 3- and 4′-hydroxyl groups to give the acyl ester derivatives of formula *2* by treatment with acylating agents using methods known in the art. The acyl ester derivatives of mycarosyltylactone are useful as intermediates in the preparation of new macrolide antibiotics.

Typical acylating agents include anhydrides, halides (usually in combination with a base or other acid scavenger) and active esters of organic acids. Acylation can also be achieved by using a mixture of an organic acid and a dehydrating agent such as N,N′-dicyclohexylcarbodiimide.

The derivatives can be prepared by esterification techniques generally known in the art, such as, for example, treatment of the compound with a stoichiometric quantity (or a slight excess) of an acylating agent, such as an acyl anhydride, in an organic solvent (for example, pyridine) at about 0°C to about room temperature for from about 1 to about 24 hours until esterification is substantially complete. The ester derivative can be isolated from the reaction mixture by standard procedures such as extraction, chromatography and crystallization.

Useful esters are those of organic acids including aliphatic, cycloaliphatic, aryl, aralkyl, heterocyclic carboxylic, sulfonic and alkoxycarbonic acids of from 1 to 18 carbon atoms, and of inorganic acids, such as sulfuric and phosphoric acids.

Preferred esters are those wherein R is an acyl moiety derived from a monocarboxylic acid or dicarboxylic acid of from 1 to 18 carbon atoms.

Representative suitable esters include those derived from acids such as formic, acetic, chloroacetic, propionic, butyric, isovaleric, glucuronic, alkoxycarbonic, stearic, cyclopropanecarboxylic, cyclohexanecarboxylic, β-cyclohexylpropionic, 1-adamantanecarboxylic, benzoic, phenylacetic, phenoxyacetic, mandelic and 2-thienylacetic acids, and alkyl-, aryl-, and aralkyl-sulfonic acids, the aryl- and aralkyl- acids optionally bearing substituents such as halogen, nitro, lower alkoxy and the like on the

* deposited 10th July 1980.

aromatic moiety. Suitable esters also include hemiesters derived from dicarboxylic acids such as succinic, maleic, fumaric, malonic and phthalic acids.

The compounds of structures *1*, *2* and *3* are useful intermediates from which 16-membered macrolide antibiotics can be prepared. For example, mycarosyltylactone (*1*) can be hydrolyzed using mild acid conditions to give tylactone (*4*). Likewise, a mycarosyltylactone derivative of formula *2* can be hydrolyzed to give the corresponding 3-O-actyltylactone derivative of formula *3*.

Mild acid hydrolysis conditions are known in the art. Appropriate solutions having a pH of about four or below can be used to accomplish the hydrolysis. A polar organic cosolvent, such as an alcohol (for example, ethanol), should be included in the solution to keep the reactants in solution. Temperatures of about 20°C to about 100°C can be used in this method. The reaction time needed to carry out the hydrolysis varies, depending upon the pH of the reaction mixture and the temperature used. At higher pH levels the reaction rate is slower, and at higher temperatures the reaction rate is faster. The reaction is carried out by treating mycarosyltylactone or a 3-O-acylmycarosyltylactone derivative with a mild acid solution for a time sufficient to effect removal of the mycarosyl group to give tylactone, or 3-O-actyltylactone.

Tylactone can be bioconverted to tylosin or tylosin-related compounds. The bioconversion is accomplished by adding tylactone to a growing culture of a bioconverting microorganism. The bioconverting microorganism can be a *Streptomyces* strain which either produces tylosin itself or is capable of producing tylosin except that it is blocked in tylactone formation.

A strain which is capable of producing tylosin except that it is blocked in tylactone formation can be obtained by treating a tylosin-producing strain which a mutagen and screening survivors for those which are unable to produce tylosin. Those survivors which are unable to produce tylosin are further screened to determine which strains are unable to produce tylactone but are still capable of bioconverting tylactone to tylosin. These strains are identified by adding tylactone to small shake-flask cultures of the selected survivors to determine if they bioconvert tylactone to tylosin.

*Streptomyces fradiae* strains NRRL 2702 and NRRL 2703 are examles of *Streptomyces* strains which are capable of producing tylosin. A typical mutagen which may be used to obtain the selected strains is N-methyl-N′-nitro-N-nitrosoguanidine.

Tylactone and the 3-O-acyltylactone derivatives are especially useful in the preparation of labelled compounds for biosynthetic or metabolic studies. By labelling either the tylactone portion or the added sugar moieties, specifically labelled tylosin useful for biosynthetic or metabolic studies can be obtained. The acyl moiety of the 3-O-acyltylactone derivatives provides an additional site for labelling.

In order to illustrate more fully the operation of this invention, the following examples are provided:

Example 1

A. Shake-flask Fermentation of Mycarosyltylactone

A lyophilized pellet of *Streptomyces fradiae* NRRL 12201 was dispersed in 1—2 ml of sterilized water. A portion of this solution (0.5 ml) was used to inoculate a vegetative medium (150 ml) having the following composition:

| Ingredient | Amount (%) |
|---|---|
| Corn steep liquor | 1.0 |
| Yeast extract | 0.5 |
| Soybean grits | 0.5 |
| $CaCO_3$ | 0.3 |
| Soybean oil (crude) | 0.45 |
| Deionized water | 97.25 |

Alternatively, a vegetative culture of *S. fradiae* NRRL 12201 preserved, in 1-ml volumes, in liquid nitrogen was rapidly thawed and used to inoculate the vegetative medium. The inoculated vegetative medium was incubated in a 500-ml Erlenmeyer flask at 29°C for about 48 hours on a closed-box shaker at about 300 rpm.

This incubated vegetative medium (0.5 ml) was used to inoculate 7 ml of a production medium having the following composition:

| Ingredient | Amount (%) |
|---|---|
| Beet molasses | 2.0 |
| Corn meal | 1.5 |
| Fish meal | 0.9 |
| Corn gluten | 0.9 |
| NaCl | 0.1 |
| $(NH_4)_2HPO_4$ | 0.04 |
| $CaCO_3$ | 0.2 |
| Soybean oil (crude) | 3.0 |
| Deionized water | 91.36 |

The inoculated fermentation medium was incubated in a 50-ml bottle at 29°C for about 6 days on a closed-box shaker at 300 rpm.

B. Tank Fermentation of Mycarosyltylactone

In order to provide a larger volume of inoculum, 60 ml of vegetative culture, prepared in a manner similar to that described in section A, was used to inoculate 38 L of a second-stage vegetative growth medium having the following composition:

| Ingredient | Amount (%) |
|---|---|
| Corn steep liquor | 1.0 |
| Soybean meal | 0.5 |
| Yeast extract | 0.5 |
| $CaCO_3$ | 0.3 |
| Soybean oil (crude) | 0.5 |
| Lecithin (crude) | 0.015 |
| Water | 97.185 |

The pH was adjusted to 8.5 with 50% NaOH solution.

This second-stage vegetative medium was incubated in a 68-liter tank for about 47 hours at 29°C.

Second-stage culture (4 L) thus prepared was used to inoculate 40 litres of sterile production medium having the following composition:

| Ingredient | Amount (%) |
|---|---|
| Fish meal | 0.92 |
| Corn meal | 1.57 |
| Corn gluten | 0.92 |
| $CaCO_3$ | 0.21 |
| NaCl | 0.10 |
| $(NH_4)_2HPO_4$ | 0.04 |
| Beet molasses | 2.10 |
| Soybean oil (crude) | 3.15 |
| Lecithin | 0.09 |
| Water | 90.90 |

The pH was adjusted to 7.2 with 50% NaOH solution.

The inoculated production medium was allowed to ferment in a 68-litre tank for about 5 days at a temperature of 28°C. The fermentation medium was aerated with sterile air to keep the dissolved oxygen level between about 30% and 50% and stirred with conventional agitators at about 300 rpm.

Example 2

Isolation of Mycarosyltylactone and Tylactone

Fermentation broth (900 ml), obtained as described in Example 1, Section A, was extracted with petroleum ether (900 ml). The petroleum ether extract was concentrated under an air stream to give an oil. The oil was dissolved in a small amount of ethyl acetate (about 15 ml). Heptane (about 15—20 ml) was added. The ethyl acetate was slowly allowed to evaporate to permit crystallization. The crystals were separated to give 450 mg of a crystalline mixture of tylactone and mycarosyltylactone.

Additional material could be obtained by adding an equal volume of methanol to the remaining whole broth, filtering the resulting solution, and extracting the filtrate with methylene chloride.

The crystalline mixture (400 mg) was separated by dissolving it in benzene. The benzene solution was chromatographed on a silica-gel (Woelm) column, packed in benzene. Elution was monitored by silica-gel thin-layer chromatography, using a benzene:ethyl acetate (3:2) solvent system and concentrated sulfuric acid spray for detection. The column was first eluted with benzene to remove lipid substances, then with one litre of benzene:ethyl acetate (9:1), 1400 ml of benzene:ethyl acetate (6:1) and 900 ml of benzene:ethyl acetate (3:1) to separate and isolate tylactone and mycarosyltylactone. Fractions having a volume of about 150 ml were collected. Tylactone was eluted first (fractions 14—19), and mycarosyltylactone later (fractions 22—26). Fractions containing each were combined, evaporated under vacuum, and crystallized from heptane to give 160 mg of tylactone and 120 mg of mycarosyltylactone.

Mycarosyltylactone is a white solid which crystallizes from heptane, hexane or ethyl acetate-hexane and which melts at about 182—184°C. It has the following approximate percentage elemental composition: carbon, 67%; hydrogen, 9%; and oxygen, 24%. It has an empirical formula of $C_{30}H_{50}O_8$ and a molecular weight of about 538

The infrared absorption spectrum of mycarosyltylactone in chloroform is shown in the accompanying drawing. Observable absorption maxima occur at the following frequencies ($cm^{-1}$): 3640 (medium), 2941 and 2907 [doublet (strong)], 2421 (very small), 1712 (strong), 1678 (medium), 1623 (small), 1590 (strong), 1456 (medium), 1404 (small), 1374 (small), 1359 (shoulder), 1314 (small), 1284 (small), 1263 (very small), 1229 (small), 1178 (strong), 1157 (medium), 1134 (very small), 1109 (small), 1078 (very small), 1050 (medium), 1025 (very small), 1000 (strong), 984 (strong), 962 (medium), 920 (very small), 911 (very small), 887 (small), 867 (small), 848 (shoulder), 836 (small), and 799 (small).

The ultraviolet absorption spectrum of mycarosyltylactone in neutral ethanol exhibits an absorption maximum at about 282 nm ($E^{1\%}_{1cm} = 568$).

Mycarosyltylactone has the following specific rotation: $[\alpha]^{25}_{D}$ −46.4° (*c* 1, $CH_3OH$).

Mycarosyltylactone is nearly insoluble in water, but is soluble in organic solvents such as acetone, methanol, ethanol, dimethylformamide, chloroform, diethyl ether, petroleum ether, benzene and dimethyl sulfoxide.

Example 3

3,4′-Di-O-acetyl-5-O-mycarosyltylactone

Mycarosyltylactone (20 mg), prepared as described in Example 2, was dissolved in pyridine (0.5 ml). Acetic anhydride (0.25 ml) was added. The resulting mixture was stirred at room temperature for 15 hours and then concentrated to dryness under vacuum. The residue was re-evaporated from methanol-cyclohexane until a solid was obtained to give 3,4′-di-O-acetyl-5-O-mycarosyltylactone.

Examples 4—7

3,4′-Di-O-propionyl-4-O-mycarosyl-tylactone, was prepared according to the procedure of Example 3, but using propionic anhydride.

3,4′-Di-O-isovaleryl-5-O-mycarosyltylactone, was prepared according to the procedure of Example 3, but using isovaleric anhydride.

3,4′-Di-O-benzoyl-5-O-mycarosyltylactone was prepared according to the procedure of Example 3, but using benzoic anhydride.

3,4′-Di-O-(*n*-butyryl)-5-O-mycarosyltylactone, was prepared according to the procedure of Example 3, but using *n*-butyric anhydride.

Example 8

Preparation of Tylactone from Mycarosyltylactone

Mycarosyltylactone, prepared as described in Example 2, was dissolved in a methanol-aqueous hydrochloric acid solution (pH 1.8). The resulting solution was allowed to stand until hydrolysis was complete (warming over a steam bath for about 30 minutes) and then adjusted to pH 7.0 by the addition of sodium hydroxide. This solution was extracted with ethyl acetate, dichloromethane or chloroform. The extract was dried and evaporated under vacuum to give tylactone.

Tylactone is a white solid which crystallizes from heptane, hexane or ethyl acetate-hexane and which melts at about 162—163°C. It has the following approximate percentage elemental composition: carbon, 70%; hydrogen, 9.7%; oxygen, 20.3%. It has an empirical formula of $C_{23}H_{38}O_5$ and a molecular weight of about 394.

The infrared absorption spectrum of tylactone in chloroform has observable absorption maxima at the following frequencies ($cm^{-1}$): 3534 (medium), 2924 (strong), 2398 (weak), 2353 (weak), 1709 (very strong), 1678 (very strong), 1626 (small), 1592 (very strong), 1458 (strong), 1441 (shoulder), 1404 (strong), 1379 (small), 1316 (strong), 1284 (medium), 1181 (very strong), 1143 (strong), 1103 (medium), 1078 (medium), 1049 (very small), 1025 (medium), 984 (very strong), 958 (strong), 923 (medium), 911 (shoulder), 859 (small), 868 (medium), 840 (medium), 820 (very small) and 661 (small).

The ultraviolet (UV) absorption spectrum of tylactone in neutral ethanol exhibits an absorption maximum at about 282 nm ($E^{1\%}_{1cm} = 560$).

Tylactone has the following specific rotation:

$$[\alpha]^{25}_{D} -55.23° \ (c\ 1,\ CH_3OH).$$

Electrometric titration of tylactone in 66% aqueous dimethylformamide indicates it has no titratable groups.

Tylactone is nearly insoluble in water, but is soluble in organic solvents such as acetone, methanol, ethanol, dimethylformamide, chloroform, diethyl ether, petroleum ether, benzene and dimethyl sulfoxide.

Example 9

Preparation of 3-O-Acetyltylactone

3,4′-Di-O-Acetyl-5-O-mycarosyltylactone, prepared as described in Example 3, was hydrolyzed according to the method of Example 8 using methanol:0.1N HCl (1:1; pH 1.5) to give 3-O-acetyltylactone.

Example 10—13

3-O-Propionyltylactone, was prepared from 3,4′-di-O-propionyl-5-O-mycarosyltylactone of Example 4 according to the procedure of Example 9.

3-O-Isovaleryltylactone, was prepared from 3,4′-di-O-isovaleryl-5-O-mycarosyltylactone of Example 5 using the procedure of Example 9.

3-O-Benzoyltylactone, was prepared from 3,4′-di-O-benzoyl-5-O-mycarosyltylactone of Example 6, using the procedure of Example 9.

3-O-(*n*-Butyryl)tylactone, was prepared from 3,4′-di-O-(*n*-butyryl)-5-O-mycarosyltylactone of Example 7, using the procedure of Example 9.

Example 14

Preparation of Tylosin from Tylactone

A *Streptomyces fradiae* strain which formerly produced tylosin but which is blocked in macrolide

ring closure was fermented according to the procedure described in Example 1, Section A. A temperature of 28°C was used. Tylactone was added to the fermentation 48 hours after inoculation. The fermentation was then continued until a substantial amount of tylosin was produced, i.e. for about three additional days. The presence of tylosin was determined by testing samples of the broth against organisms known to be sensitive to tylosin. One useful assay organism was *Staphylococcus aureus* ATCC 9144. Bioassay was conveniently performed by an automated turbidometric method. Alternative assay methods include thin-layer chromatography and high-performance liquid chromatography with UV detection.

Example 15

Preparation of Labelled Tylosin

Mycarosyltylactone was prepared by the method of Examples 1 and 2 except that a labelled acetate, propionate, or butyrate was incorporated into the fermentation medium. Labelled mycarosyltylactone thus produced was used to prepare labelled tylactone using the method of Example 8. The labelled tylactone was used to prepare tylosin according to the procedure of Example 14. Tylosin labelled on the macrolide ring was thereby provided.

Example 16

Preparation of 3-O-Acetyl-5-O-$\beta$-desosaminyltylactone

3-O-Acetyltylactone, prepared as described in Example 10, was mixed with 1-$\alpha$-bromo-2-O-acetyldesosamine hydrobromide (5-equivalents) in the presence of mercuric cyanide in nitromethane (20°C, 10 hours). 3,2'-O-diacetyl-5-O-$\beta$-desosaminylactone was isolated using silica gel column chromatography. The 2'-acetyl group was removed by allowing this compound to stand in methanol at room temperature to give 3-O-acetyl-5-O-$\beta$-desosaminyltylactone (the 3-O-acetyl derivative of antibiotic M—4365 $G_1$).

**Claims for the Contracting States: BE CH DE FR GB IT LI LU NL SE**

1. A macrolide of formula (I):

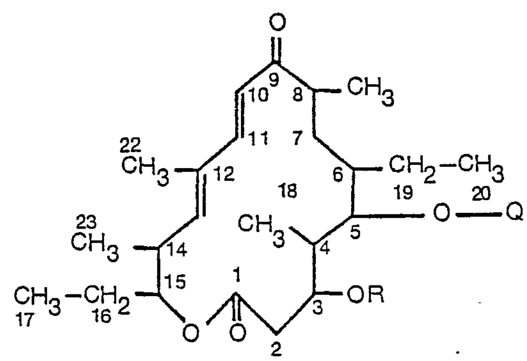

(I)

wherein R is hydrogen or acyl, and wherein Q is hydrogen or a mycarosyl group of formula:

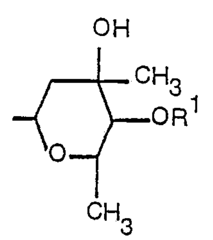

wherein $R^1$ is hydrogen or acyl;
provided that when Q is hydrogen, R is acyl.

2. A macrolide of formula (I) as claimed in claim 1, wherein when R is acyl, Q is hydrogen or a mycarosyl group in which $R^1$ is acyl.

3. A macrolide of formula (I) as claimed in claim 1 or 2, where R and $R^1$ are acyl moieties derived from a mono or dicarboxylic acid containing from 1 to 18 carbon atoms.

4. Mycarosyltylactone.

5. A process for preparing a macrolide of formula (I) which comprises culturing a strain of *Streptomyces fradiae* NRRL 12201 under submerged aerobic conditions in a culture medium containing assimilable sources of carbon, nitrogen and inorganic salts so as to produce a compound of formula (I) in which R is hydrogen and Q is a mycarosyl group in which $R^1$ is hydrogen; followed optionally by:

(a) acylation to form a compound of formula (I) in which one or both of R and Q are acyl;

(b) mild hydrolysis of a compound of formula (I) prepared as in (a) above to give a macrolide of formula (I) in which Q is hydrogen and R is acyl.

**Claims for the Contracting State: AT**

1. A process for preparing a macrolide of formula (I):

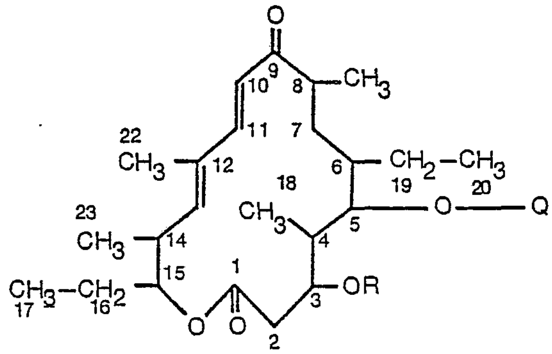

(I)

wherein R is hydrogen or acyl, and wherein Q is hydrogen or a mycarosyl group of formula:

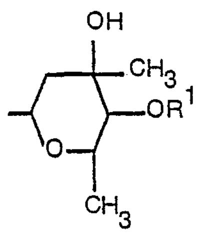

wherein $R^1$ is hydrogen or acyl;
provided that when Q is hydrogen, R is acyl;
which comprises culturing a strain of *Streptomyces fradiae* NRRL 12201 under submerged aerobic conditions in a culture medium containing assimilable sources of carbon, nitrogen and inorganic salts so as to produce a compound of formula (I) in which R is hydrogen and Q is a mycarosyl group in which $R^1$ is hydrogen; followed optionally by:

(a) acylation to form a compound of formula (I) in which one or both of R and Q are acyl;

(b) mild hydrolysis of a compound of formula (I) prepared as in (a) above to give a macrolide of formula (I) in which Q is hydrogen and R is acyl.

2. A process according to claim 1 for preparing mycarosyltylactone.

**Revendications pour les Etats contractants: BE CH DE FR GB IT LI LU NL SE**

1. Macrolide de formule (I):

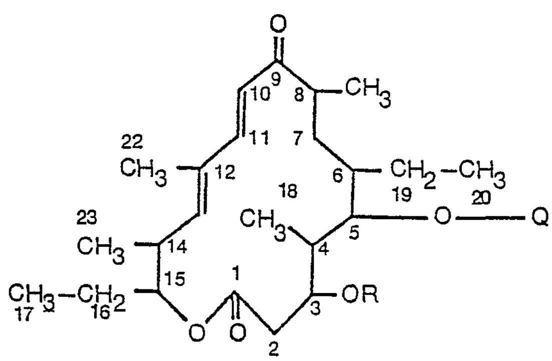

(I)

dans laquelle R représente un atome d'hydrogène ou un groupe acyle, tandis que Q représente un atome d'hydrogène ou un groupe mycarosyle de formule:

**Revendications pour l'Etat contractant: AT**

1. Procédé de préparation d'un macrolide de formule (I):

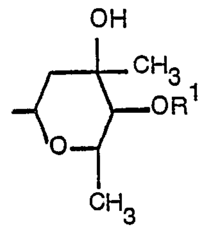

dans laquelle $R^1$ représente un atome d'hydrogène ou un groupe acyle; avec cette réserve que si Q est un atome d'hydrogène, R est un groupe acyle.

2. Macrolide de formule (I) suivant la revendication 1, dans lequel, lorsque R est un groupe acyle, Q est un atome d'hydrogène ou un groupe mycarosyle dans lequel $R^1$ est un groupe acyle.

3. Macrolide de formule (I) suivant la revendication 1 ou 2, dans lequel R et $R^1$ sont de fractions acyle dérivant d'uin acide mono- ou dicarboxylique contenant 1 à 18 atomes de carbone.

4. La mycarosyltylactone.

5. Procédé de préparation d'un macrolide de formule (I), caractérisé en ce qu'il consiste à cultiver une souche de *Streptomyces fradiae* NRRL 12201 dans des conditions aérobies submergées et dans un milieu de culture contenant des sources assimilables de carbone, d'azote et de sels inorganiques de façon à obtenir un composé de formule (I) dans laquelle R est un atome d'hydrogène et Q est un groupe mycarosyle dans lequel $R^1$ est un atome d'hydrogène, cette étape étant facultativement suivie de:

(a) une acylation pour former un composé de formule (I) dans laquelle un ou les deux radicaux R et Q sont des groupes acyle;

(b) une hydrolyse modérée d'un composé de formule (I) préparé comme décrit sub (a) ci-dessus pour obtenir un macrolide de formule (I) dans laquelle Q est un atome d'hydrogène et R est un groupe acyle.

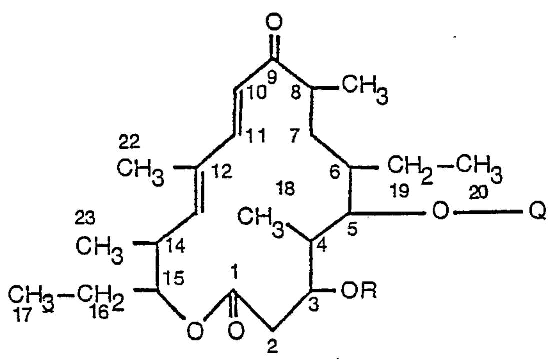

(I)

dans laquelle R est un atome d'hydrogène ou un groupe acyle, tandis que Q est un atome d'hydrogène ou un groupe mycarosyle de formule:

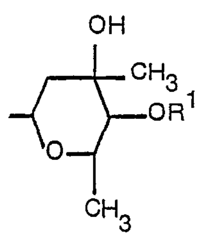

dans laquelle $R^1$ est un atome d'hydrogène ou un groupe acyle; avec cette réserve que si Q est un atome d'hydrogène, R est un groupe acyle; caractérisé en ce qu'il consiste à cultiver une souche de *Streptomyces fradiae* NRRL 12201 dans des conditions aérobies submergées et dans un milieu de culture contenant des sources assimilables de

carbone, d'azote et de sels inorganiques pour obtenir un composé de formule (I) dans laquelle R est un atome d'hydrogène et Q est un groupe mycarosyle dans lequel $R^1$ est un atome d'hydrogène; cette étape étant facultativement suivie de:

(a) une acylation pour former un composé de formule (I) dans laquelle un ou les deux radicaux R et Q sont des groupes acyle;

(b) une hydrolyse modérée d'un composé de formule (I) préparé comme décrit sub (a) ci-dessus pour obtenir un macrolide de formule (I) dans laquelle Q est un atome d'hydrogène et R est un groupe acyle.

2. Procédé suivant la revendication 1 pour la préparation de la mycarosyltylactone.

**Patentansprüche für die Vertragsstaaten: BE CH DE FR GB IT LI LU NL SE**

1. Makrolid der Formel (I)

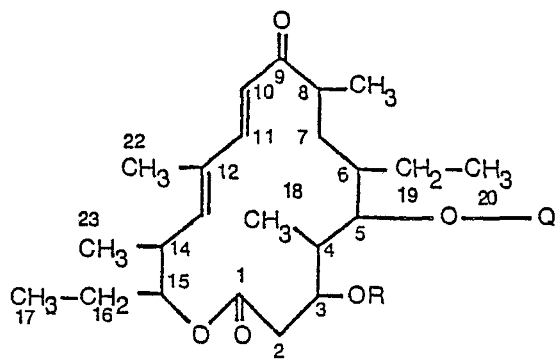

(I)

worin R Wasserstoff oder Acyl ist und worin Q Wasserstoff oder eine Mycarosylgruppe der Formel

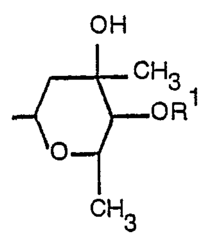

ist, wobei $R^1$ Wasserstoff oder Acyl bedeutet, mit der Maßgabe, daß R für Acyl steht, falls Q Wasserstoff ist.

2. Makrolid der Formel (I) nach Anspruch 1, dadurch gekennzeichnet, daß Q Wasserstoff oder eine Mycarosylgruppe ist, worin $R^1$ für Acyl steht, falls R Acyl ist.

3. Makrolid der Formel (I) nach Anspruch 1 oder 2, dadurch gekennzeichnet, daß R und $R^1$ Acylreste sind, die von einer Mono- oder Dicarbonsäure abgeleitet sind, die 1 bis 18 Kohlenstoffatome enthält.

4. Mycarosyltylacton.

5. Verfahren zur Herstellung eines Makrolids der Formel (I), dadurch gekennzeichnet, daß man einen Stamm von Streptomyces fradiae NRRL 12201 unter submersen aeroben Bedingungen in einem Kulturmedium, das assimilierbare Quellen für Kohlenstoff, Stickstoff und anorganische Salze enthält, zur Bildung einer Verbindung der Formel (I), worin R Wasserstoff ist und Q für einen Mycarosylrest steht, worin $R^1$ Wasserstoff ist, züchtet und gegebenenfalls

(a) durch Acylierung eine Verbindung der Formel (I) bildet, worin einer oder beide Reste R und Q Acyl bedeuten, oder

(b) eine gemaß obiger Stufe (a) hergestellte Verbindung der Formel (I) durch Hydrolyse unter milden Bedingungen in ein Makrolid der Formel (I) überführt, worin Q Wasserstoff ist und R für Acyl steht.

**Patentansprüche für den Vertragsstaat: AT**

1. Verfahren zur Herstellung eines Makrolids der Formel (I)

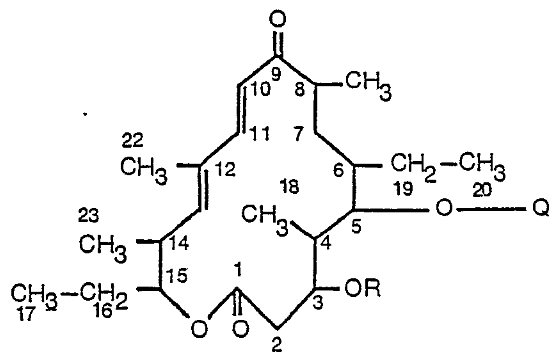

(I)

worin R Wasserstoff oder Acyl ist und worin Q Wasserstoff oder eine Mycarosylgruppe der Formel

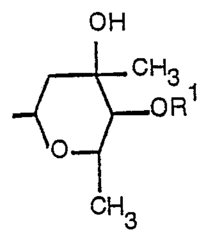

ist, wobei $R^1$ Wasserstoff oder Acyl bedeutet, mit der Maßgabe, daß R für Acyl steht, falls Q Wasserstoff ist, dadurch gekennzeichnet, daß man einen Stamm von Streptomyces fradiae NRRL 12201 unter submersen aeroben Bedingungen in einem Kulturmedium, das assimilierbare Quellen für Kohlenstoff, Stickstoff und anorganische Salze enthält, zur Bildung einer Verbindung der Formel (I), worin R Wasserstoff ist und Q für einen Mycarosylrest steht, worin $R^1$ Wasserstoff ist, züchtet und gegebenenfalls

(a) durch Acylierung eine Verbindung der Formel (I) bildet, worin einer oder beide Reste R und Q Acyl bedeuten, oder

(b) eine gemäß obiger Stufe (a) hergestellte Verbindung der Formel (I) durch Hydrolyse unter milden Bedingungen in ein Makrolid der Formel (I) überführt, worin Q Wasserstoff ist und R für Acyl steht.

2. Verfahren nach Anspruch 1, dadurch gekennzeichnet, daß man Mycarosyltylacton herstellt.

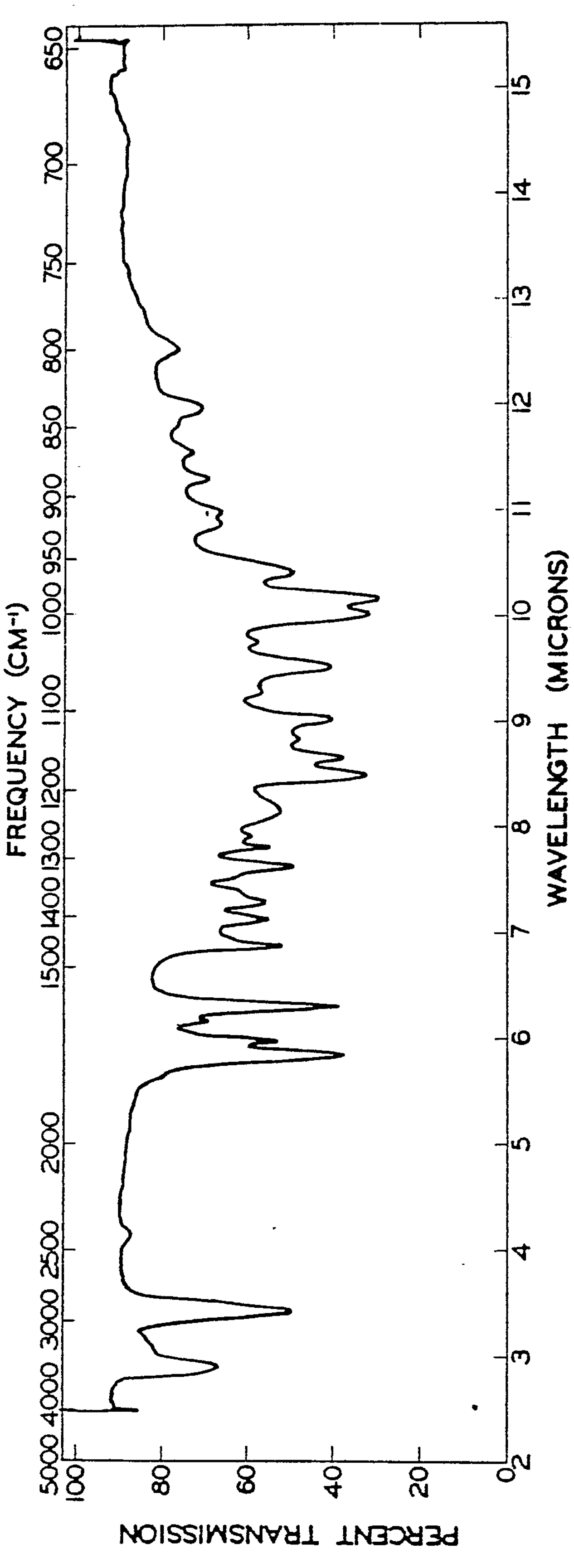
FREQUENCY (CM⁻¹)
5000 4000 3000 2500 2000 1500 1400 1300 1200 1100 1000 950 900 850 800 750 700 650
WAVELENGTH (MICRONS)
2 3 4 5 6 7 8 9 10 11 12 13 14 15
PERCENT TRANSMISSION
0 20 40 60 80 100